**Europäisches Patentamt**

**European Patent Office**

**Office européen des brevets**

(19)

(11) Veröffentlichungsnummer : **0 186 799 B2**

(12) # NEUE EUROPÄISCHE PATENTSCHRIFT

(45) Veröffentlichungstag der neuen Patentschrift :
20.10.93 Patentblatt 93/42

(51) Int. Cl.⁵ : **G01N 33/52, G01N 33/558**

(21) Anmeldenummer : **85115333.8**

(22) Anmeldetag : **03.12.85**

(54) **Flächenförmiges diagnostisches Mittel.**

(30) Priorität : **15.12.84 DE 3445816**

(43) Veröffentlichungstag der Anmeldung :
**09.07.86 Patentblatt 86/28**

(45) Bekanntmachung des Hinweises auf die
Patenterteilung :
**21.02.90 Patentblatt 90/08**

(45) Bekanntmachung des Hinweises auf die
Entscheidung über den Einspruch :
**20.10.93 Patentblatt 93/42**

(84) Benannte Vertragsstaaten :
**AT BE CH DE FR GB IT LI LU NL SE**

(56) Entgegenhaltungen :
**EP-A- 0 046 004**
**EP-A- 0 052 328**
**EP-A- 0 100 619**
**FR-A- 2 191 734**
**US-A- 3 847 553**
**US-A- 4 361 537**
**US-A- 4 446 232**
**US-A- 4 459 358**

(73) Patentinhaber : **BEHRINGWERKE**
**Aktiengesellschaft**
**Postfach 1140**
**D-35001 Marburg (DE)**

(72) Erfinder : **Friesen, Heinz-Jürgen, Dr.**
**Im Dorf 4**
**D-3550 Marburg 21 (DE)**
Erfinder : **Grenner, Gerd, Dr.**
**Höhenweg 72**
**D-3550 Marburg 1 (DE)**
Erfinder : **Pauly, Hans-Erwin, Dr.**
**Finkenstr. 1**
**D-3563 Dautphetal 2 (DE)**
Erfinder : **Kohl, Helmut, Dr.**
**Goethestr. 32**
**D-3552 Wetter (DE)**
Erfinder : **Habenstein, Klaus, Dr.**
**Im Ketzergrund 33**
**D-3552 Wetter (DE)**
Erfinder : **Stärk, Joseph, Dr.**
**Im Winkel 2**
**D-3557 Ebsdorfergrund 8 (DE)**

**Beschreibung**

Die Erfindung betrifft ein festes diagnostisches Mittel, bestehend aus mehreren Funktionsfeldern, welches zum Nachweis und zur quantitativen Bestimmung von Substanzen oder Analyten in biologischen Flüssigkeiten dient. Die Erfindung betrifft weiterhin ein Verfahren unter Verwendung dieses Mittels, wobei nach Kontakt des Mittels mit der Flüssigkeit die Analyten mit spezifischen bioaffinen Bindungspartnern reagieren und mit Hilfe von Markierungsreagenzien nachgewiesen werden.

In der Diagnostik hat die Fähigkeit, spezifische Verbindungen zu identifizieren und zu bestimmen, die Überwachung von Medikamentengaben, Ouantifizierung von physiologisch aktiven Verbindungen oder ihrer Folgeprodukte sowie Diagnose von Infektionen ermöglicht. Den Immunoassay-Methoden (RIA, ELISA, Agglutinationstest) kommt dabei eine besondere Bedeutung zu. Die bei den Tests ausgenutzten spezifischen Bindungsreaktionen beschränken sich nicht auf immunologische Interaktionen, wie Antigen bzw. Hapten-Antikörper-Wechselwirkungen, sondern beutzen auch bioaffine Wechselwirkungen wie Lectin-Zucker, Wirkstoff-Rezeptor.

Vorhandene Tests sind zwar empfindlich und spezifisch, jedoch aufgrund der langen Testdauer (meist mehrere Stunden oder sogar Tage) und der häufigen Testschritte, wie Immunreaktion, Waschschritte, enzymatische Reaktion, stellen sie keine bequemen Anwendungsformen dar. Die langen Testzeiten sind mit dem Einsatz in der Notfalldiagnostik nicht verträglich.

Integrierte trockenchemische Testelemente, wie sie in der vorgestellten Erfindung beschrieben werden, vereinfachen die Testdurchführung und verkürzen die Testzeiten.

Testelemente, bestehend aus übereinandergeschichteten Funktionszonen, die zum Nachweis von Analyten mit bioaffinen Bindungseigenschaften dienen, sind bereits bekannt. In EP-A-0 097 952 und in DE-A-3 329 728 sind solche beschrieben, bei denen sich das signalproduzierende System (SPS) auf die Fluoreszenzmarkierung eines der bioaffinen Reaktions- oder Bindungspartner beschränkt. In US-A-4 446 232 und in US-A-4 472 498 sind außerdem Testelemente beschrieben, die zum einen als SPS Markierungsenzym und chromogenes Substrat enthalten und zum anderen den Nachweis des Analyten in frei beweglichen, nicht an eine Festphase gebundenen Komplexen vorsehen.

Testelemente mit nebeneinander angeordneten, flächenförmigen Funktionsbereichen sind nach Art eines Teststreifens in US-A-4 366 241 oder dem Äquivalent EP-A-0 046 004 beschrieben. Hier ist einer der bioaffinen Reaktionspartner in einem ersten, am einen Ende des Teststreifens liegenden Funktionsbereich als Festphase gebunden. Bei Kontakt der Lösung des Analyten mit diesem ersten Funktionsbereich wird der Analyt dort gebunden. Das Testelement muß zuerst an genanntem ersten Funktionsbereich mit der Lösung des Analyten, anschließend mit einer Lösung des markierten, in der Spezifität von dem Festphasenreaktionspartner verschiedenen Reaktionspartner des Analyten und im Falle einer Enzymmarkierung in einem dritten Schritt mit einer Lösung von chromogenem Substrat in Berührung gebracht werden.

Es wurde überraschenderweise gefunden, daß ein trockenes, alle Reagenzkomponenten enthaltendes, flächenförmiges diagnostisches Mittel herstellbar ist, mit dem Analyten mit bioaffinen Eigenschaften nachgewiesen werden können.

Gegenstand der Erfindung ist ein analytisches Mittel zum Nachweis oder zur Bestimmung einer Komponente eines bioaffinen Bindungspaares (Analyt) in einer Flüssigkeit, bestehend aus mehreren flächenförmigen Zonen, die so hintereinander angeordnet sind, daß sie über ihre Kanten miteinander in saugfähigem Kontakt stehen, und daß sie zusammen mit der festen Unterlage ein flächenförmiges chromatographisches analytisches Mittel darstellen, enthaltend eine Elutionsmittelauftragszone (EMAZ) am einen Ende und eine Saugzone (SZ) am anderen Ende des Mittels sowie dazwischenliegende weitere saugfähige Zonen, in denen zu bioaffinen Wechselwirkungen mit dem Analyten befähigte Reaktionsteilnehmer so angeordnet sind, daß miteinander reaktionsfähige Reaktionsteilnehmer räumlich getrennt vorliegen, wobei ein Reaktand kovalent oder adsorptiv über eine bioaffine Wechselwirkung in einer zwischen der EMAZ und SZ und mit der SZ in Kontakt befindlichen Zone, der Festphasenzone (FPZ), fixiert ist oder in einer im Mittel ablaufenden Reaktion durch einen kovalent oder adsorptiv oder über eine bioaffine Wechselwirkung in der FPZ fixierten weiteren Reaktanden gebunden wird und wobei die Analytauftragzone die EMAZ oder eine Zone zwischen EMAZ und SZ ist, und ein markierter Reaktand sich in einer Zone zwischen der EMAX und FPZ nicht gebunden befindet, wobei die flächenförmigen Zonen aus Streifen gebildet sind, die aus unterschiedliche Materialien bestehen, wobei jeder Streifen auf der festen Unterlage fixiert ist.

Ein zweiter oder weitere Analyten als Bestandteil derselben Lösung können mit dem Mittel gleichzeitig nachgewiesen werden, wenn diese von dem ersten verschiedene bioaffine Eigenschaften besitzen. Sie werden ebenfalls in einem einzigen Funktionsbereich, einer für sie zuständigen Festphasenzone, in gleicher Weise wie der erste Analyt nachgewiesen. Die Funktionsbereiche für den Nachweis des zweiten oder weiterer Analyten liegen auf dem flächenförmigen Mittel vor oder hinter dem Funktionsbereich für den Nachweis des ersten Analyten. Das Mittel kann auch mehrere Festphasenzonen, die für einen Analyten und unterschiedliche Meßbereiche dieses Analyten zuständig sind, enthalten. Das Mittel und seine Komponenten liegen in trockener Form vor. Das flächenförmige diagnostische Mittel besteht aus einem oder auch aus mehreren, hintereinander angesordneten Streifen aus unterschiedlichen Materialien, die für wäßrige Lösungen saugfähig sind.

Die Streifen sind auf einer festen Unterlage fixiert. Sie enthalten die für das jeweilige diagnostische Mittel notwendigen Reagenzkomponenten und werden somit zu Funktionsfeldern oder Funktionsbereichen. Das an einem Ende des streifenförmigen Mittels liegende Funktionsfeld (Lösungsmittelauftragzone) wird mit Analytlösung durch Eintauchen in diese oder durch Auftragen derselben in Berührung gebracht. Die Lösung durchwandert alle Funktionsbereiche. Die Saugfähigkeit der Trägermaterialien, aus denen die Streifen bestehen, verursacht einen Flüssigkeitsstrom, der am anderen Ende des streifenförmigen Mittels zum Stillstand kommt. Der Analyt kann auch im mittleren Bereich des Mittels aufgebracht und anschließend ein Flüssigkeitstrom vom einen zum anderen Ende des Mittels erzeugt werden.

Die Probe muß dabei nicht direkt auf den chromatographierenden Teil des Teststreifens aufgegeben werden. Sie kann auch auf ein saugfähiges Material, das auf den Teststreifen aufliegt und das die Funktion hat, Blutzellen aus der Probe zu entfernen, aufgetragen werden. Die Probe gelangt dann nach Filtration auf den Teststreifen. Bei diesem Filtrationsprozeß kann gleichzeitig eine Zugabe von Reagenzien durch Herauslösen von in dem Filter trocken vorliegenden Komponenten erfolgen. Durch solche Komponenten können Störfaktoren aus der Lösung beseitigt werden. So kann etwa durch ein geeignetes Oxidationsmittel die in einer Probe enthaltene Ascorbinsäure, die bei Verwendung von Oxidasen und Peroxidasen als Markierungsmittel stört, unschädlich gemacht werden. Desweiteren kann das Filter auch die Funktion eines Adsorbens haben, das Störfaktoren durch Adsorption aus der Probe entfernt. Filtrations-, Adsorbens- und Reagenzzumischfunktion zur Probenkonditionierung für den Test kann auch von der Elutionsmittelauftragzone oder einer dahinterliegenden Zone übernommen werden.

Die Verteilung des Lösungsmittels in den einzelnen Funktionsbereichen ist abhängig von der Saugfähigkeit und der Dimensionierung der verwendeten Materialien.

Die Lösungsmittelauftragzone kann die Funktion eines Volumendosierelementes haben, wie in DE-A-3 043 608, DE-A-2 332 760, US-A-3 464 560, US-A-3 600 306, US-A-3 677 607, US-A-3 902 847, US-A-4 144 306 und US-A-4 258 001 beschrieben wurde. Es kann die verschiedenen, für die Funktion des Testelementes erforderlichen Reagenzien in Trockenform enthalten. Die Lösungsmittelauftragzone kann ein Stück Vliespapier sein, das sich an einem Ende des Testelementes befindet und das sich durch bloßes Eintauchen in eine Lösung, z.B. die der Probe oder durch kurzes Überspülen mit Leitungswasser mit einem definierten Flüssigkeitsvolumen vollsaugt und anschließend die Flüssigkeit langsamer und in kontrollierter Weise an die folgenden Zonen weitergibt. Die Lösungsmittelsauftragzone ist so dimensioniert, daß sie genügend Flüssigkeit hergibt, um diese zum anderen Ende des Mittels, dem Ende der Saugzone wandern zu lassen.

Zwischen Lösungsmittelauftragszone und Saugzone befinden sich die Funktionsbereiche, in denen Reaktionskomponenten für den Testablauf enthalten sind und in denen alle Reaktionsschritte des Testablaufes stattfinden. Ein Teil der Reaktionskomponenten für den Testablauf kann auch in der Probenauftragszone untergebracht sein. Die Saugzone hat die Aufgabe, überschüssige, freibewegliche Reagenzkomponenten und Reaktionsprodukte des signalproduzierenden Systems aufzunehmen.

Die saugfähigen Trägermaterialien in Form von einem oder mehreren Streifen als Bestandteile der verschiedenen Funktionsbereiche können wahlweise aus Cellulose, aus chemisch derivatisierter Cellulose oder aus Kunststoff poröser oder faseriger Struktur mit hinreichend hydrophilen Eigenschaften wie auch aus Kunststoffmembran-eingebetteten Partikeln wie Cellulose oder Silicagel und weiterhin aus hydrophilen, aber wasserunlöslich gemachten Naturprodukten bestehen. Eine Kombination von Streifen, bestehend aus verschiedenen Materialien kann verwendet werden. Die Auswahl von geeigneten saugfähigen Materialien erfolgt gemäß den Anforderungen an das jeweilige diagnostische Mittel.

Die bioaffinen Reagenzkomponenten sind bei verschiedenen Ausführungsformen von immunchemischen, diagnostischen Mitteln Antigene, Haptene oder Antikörper. Im Falle des Nachweises von Glycoproteinen oder Oligosacchariden, die sich an Lectine binden, kann ein bioaffiner Reaktionspartner das spezifische Lectin sein, der zweite bioaffine Reaktionspartner ein Antikörper, der gegen eines von dem Lectin verschiedene Bindungsstelle des Analyten gerichtet ist. Im Falle des Nachweises von mikrobiellen Wirkstoffen kann der eine Bindungspartner die Rezeptorsubstanz für den Wirkstoff, der zweite Bindungspartner ein Antikörper gerichtet gegen eine andere Bindungsstelle des Wirkstoffs, sein.

Ein bioaffiner Bindungspartner wird während des Reaktionsablaufs oder ist schon vorher an das Trägermaterial in dem Funktionsbereich gebunden, der zum Nachweis des Analyten vorgesehen ist (Festphasenzone). Er wird auch Festphasen-Bindungspartner genannt. Der oder die übrigen Bindungspartner sind in den Trägermaterialien enthalten. Sie sind mit einer Markierung versehen.

Von den verschiedenen, bekannten Markierungsmöglichkeiten wird die Enzymmarkierung bevorzugt. Sie

3

erfordert chromogene, Fluoreszenz oder Chemilumineszenz erzeugende Substratsysteme. Die Chemilumineszenzmarkierung stellt ein weiteres Beispiel einer Markierung, die erst nach Zugabe eines Reagenzes gemessen wird, dar. Gemessen werden kann dabei die Chemilumineszenz selbst oder eine Fluoreszenz, die durch sie angeregt wird. Die Fluoreszenzmarkierung wird meist gemessen, ohne daß die Zugabe eines Reagenzes erforderlich ist. Es kann aber auch wie bei der Verwerndung bestimmter Seltenerdchelate erwünscht sein, den zu messenden Fluorophor erst durch Zugabe eines Reagenzes zu erzeugen oder einen zweiten Fluorophor zuzugeben, der durch den ersten angeregt wird oder den ersten anregt. Die Fluoreszenz kann einfach, zeitaufgelöst oder als Fluoreszenzpolarisation gemessen werden.

Ein zum Nachweis benötigtes Reagenz kann nach dem Trennschritt mit dem nachzuweisenden Immunokomplex auf verschiedene Weise in Reaktion gebracht werden. Ein Teil des signalproduzierenden Systems kann in der Festphasenzone vorkommen. Ein zum Nachweis der Markierung benötigtes Reagenz kann nach genügendem Waschen der Festphase beim heterogenen Immunoassay mit Detektion in der gebundenen Phase in verschiedenen Ausführungsformen verzögert aufgegeben werden. Möglichkeiten sind z.B.:

Aufbringen von Reagenzien durch einen zum Hauptflüssigkeitsstrom parallel geschalteten, langsamer fließenden Flüssigkeitsstrom, welcher vom Elutionsmittelreservoir ausgeht und vor der Zone mit der markierten Komponente einmündet. Der parallel geschaltete Flüssigkeitsstrom kann gesteuert werden durch Verwendung eines langsamer chromatographierenden saugfähigen Mediums, etwa eines entsprechend langsam chromatographierenden Papiers oder eines Papiers, das stellenweise mit « den Weg vorübergehend blockierenden Komponenten », wie z.B. beim Auflösen eine hohe Viskosität verleihende Polymere (z.B. Polyvinylalkohole, Dextrane), imprägniert ist.

Das Aufbringen von Reagenzien kann nach genügendem Waschen der Festphase (= Beendigung der Chromatographie) durch Niederdrücken eines Elementes, das fester Bestandteil des Testelementes ist, erfolgen. Das « Niederdrücken » kann mechanisch oder durch Entfernen von Abstandshaltern durch Einwirkung des Flüssigkeitsstromes erfolgen. Beispielsweise kann das mechanische Niederdrücken eines die Reagenzien enthaltenden Elementes durch Niederdrücken einer Klappe oder eines durch Abstandshalter gehaltenen Papiers erfolgen. Das Herabsenken eines die Reagenzien enthaltenden Elementes unter Einwirkung des Flüssigkeitsstromes kann z.B. erreicht werden durch Übereinanderlaminieren von Festphase, eines wasserlöslichen Polymeren und des Reagenzträgers (z.B. ein ensprechend imprägniertes Papier).

Ein verzögertes Einbringen von Reagenzien in den Flüssigkeitsstrom kann geschehen unter Verwendung von mikroverkapseltem Reagenz, das erst nach genügendem Waschen der Festphase aus der Verkapselung austritt oder durch Überziehen des in der Matrix anhaftenden Reagenzes mit sich langsam auflösenden Komponenten.

Eine für den Sonderfall der Enzymmarkierung dargestellte Möglichkeit sieht wie folgt aus: Bei Verwendung einer Peroxidasemarkierung kann vor die Festphasenzone eine Glucoseoxidasezone gesetzt werden. In den Flüssigkeitsstrom wird dann Glucose und auch das Chromogen mit inkorporiert, was zur Farbbildung hinter der Glucoseoxidase führen kann. Wesentliche Farbbildung wird erst beobachtet, wenn bei entsprechend hoher Peroxidasekonzentration genügend $H_2O_2$ durch die Oxidase gebildet wird. Diese Bildung des Peroxyds setzt langsam ein, erreicht eine optimale Konzentration und schließlich eine hohe Konzentration, die zu einer Inhibierung des Enzyms und damit automatischem Stoppen der Farbbildung führt. Diese Färbung kann moderiert werden, wenn in die Oxidasezone oder davor ein $H_2O_2$-Fänger z.B. ein Thioether als mildes Reduktionsmittel oder das Enzym Katalase inkorporiert werden.

Es wird bei diesem Beispiel durch eine Verzögerungsschaltung unter Zuhilfenahme eines Enzyms ein Reagenz zum Nachweis der Markierung erzeugt. Die Farbbildung in der Festphasenzone setzt erst ein, nachdem diese Zone durch den Flüssigkeitsstrom von nicht spezifisch gebundener Markierung genügend freigewaschen wurde.

Zur Herstellung der Festphasenzone gibt es mehrere Möglichkeiten. Die dort fixierten Komponenten können chemisch kovalent oder adsorptiv an einen saugfähigen Träger gebunden sein, der ein Teil des Testelementes ist. Diese Komponenten können auch an eine Partikeldispersion, die nach Auftrag auf einen saugfähigen Träger am Auftragsort fixiert bleibt, gebunden sein. Beispielsweise sind Suspensionen von Zellen, die auf der Oberfläche spezifische Rezeptoren tragen, wie etwa Staphylococcus aureus Cowan I-Zellen oder Latexpartikel, die auf der Oberfläche bioaffine Bindungspartner gebunden tragen, geeignet, in einer Papiermatrix fixiert zu werden. Die an pipettierbare Träger gebundenen wie auch die nicht gebundenen Komponenten des Teststreifens können auf die saugfähige Matrix des Elementes durch Lufttrocknung eingetrocknet werden; Gefriertrocknungsschritte sind nicht unbedingt erforderlich.

Einige Testabläufe seien beispielhaft als Ausführungsformen dargestellt, die unabhängig von der verwendeten Markierung zu sehen sind. Sie sind der Einfachheit halber nur für den Nachweis eines einzigen Analyten mit dem diagnostischen Mittel beschrieben.

Für den Fall, daß der Analyt nur eine einzige bioaffine Bindungsstelle besitzt oder nur eine bioaffine Bindungsstelle von mehreren ausgenutzt wird, seien folgende zwei Ausführungsformen, die dem Prinzip des kompetitiven Immunoassays entsprechen, dargestellt:

Der Festphasen-Bindungspartner ist kovalent oder adsorptiv an das Trägermaterial des Festphasen-Funktionsbereichs gebunden. Die Lösung des Analyten macht eine dem in dem diagnostischen Mittel enthaltene vorgegebene Menge von markiertem Analyt beweglich. Beide Komponenten wandern in das Funktionsfeld, welches den Festphasen-Bindungspartner enthält und konkurrieren um die Bindung mit dem Festphasen-Bindungspartner. Ist der Anteil des Analyten gegenüber dem markierten Analyten hoch, wird wenig markierter Analyt gebunden. Ist er niedrig, wird viel markierter Analyt gebunden.

Der Festphasen-Bindungspartner ist in einem Funktionsbereich vor dem Festphasen-Funktionsbereich als nicht gebundene Komponente untergebracht. Die ankommende Lösungsmittelfront transportiert ihn in den Festphasen-Funktionsbereich, wo er gebunden wird. Diese Festphasenbindung wird durch bioaffine Bindungssysteme erzeugt, die unabhängig von dem Bindungssystem des Analyten sind. Ein mit Biotin konjugierter Bindungspartner bindet sich an trägergebundenes Avidin. Ein Immunglobulin, wie IgG als Bindungspartner wird über sein Fc-Teil an trägergebundenes Protein A von S. aureus fixiert oder von einem träger-gebundenen, nicht idiotypischen Antikörper gebunden.

Analyt und markierter Analyt konkurrieren als Bestandteil des diagnostischen Mittels während des Funktionsablaufs um die Bindungen an den Festphasen-Bindungspartner, wie zuvor beschrieben. Diese Konkurrenzreaktion spielt sich teilweise mit dem gelösten und teilweise mit dem schon festphasengebundenen Festphasen-Bindungspartner ab.

Sind bei einem Analyten zwei Bindungsstellen mit unterschiedlicher Spezifität vorhanden, sind mehrere Ausführungsformen des diagnostischen Mittels denkbar, die dem Prinzip des Sandwich-Immunoassays entsprechen. Von diesen seien ebenfalls zwei im folgenden dargestellt:

Ist der Festphasen-Bindungspartner kovalent oder adsorptiv an das Trägermaterial des Festphasen-Funktionsbereichs gebunden, bildet der Analyt mit dem markierten Bindungspartner einen binären Komplex, der mit dem Lösungsmittel in den Festphasen-Funktionsbereich hineinwandert und dort mit dem Festphasen-Bindungspartner reagiert, wobei sich ein ternärer an der Festphase gebundener Komplex ausbildet, der über die Markierung des ersten Bindungspartners nachweisbar ist. Der überschüssige markierte Bindungspartner wird durch das Lösungsmittel in den sich anschließenden Funktionsbereich, in die Saugzone abgeführt.

Wenn der Festphasen-Bindungspartner in dem diagnostischen Mittel in nicht gebundener Form vorliegt und durch das Lösungsmittel beweglich wird, werden die beiden bioaffinen Reaktionspartner des Analyten in den Funktionsbereichen so untergebracht, daß der Analyt mit beiden Partnern gleichzeitig oder nacheinander reagiert und daß der gebildete ternäre Komplex anschließend in den Festphasen-Funktionsbereich wandert, wo er, wie schon zuvor beschrieben, über ein zweites, von dem des Analyten unbhängiges, bioaffines System an die Festphase gebunden wird.

Zur Veranschaulichung der zuvor beschriebenen und weiterer Ausführungsformen, die dem immunometrischen Testprinzip, dem Prinizip des indirekt Antikörpernachweises oder dem ELA-Prinzip (Enzyme-Labelled-Antigen) des Immunoassays entsprechen, sind Verteilung der Komponenten des Mittels in den Funktionsbereichen sowie nach Reaktionsablauf die Zusammensetzung des Festphasenkomplexes, dessen Menge ein Maß für die Analytenkonzentration in der Probe ist, beispielhaft in den Übersichten I und II dargestellt.

Es wurde gefunden, daß ein voll integrierter Teststreifen, der nach dem Prinzip des heterogenen Immunoassays mit Festphasendetektion arbeitet, nicht nur prinzipiell machbar, sondern auch noch innerhalb eines Zeitraums von weniger als einer Stunde auswertbar ist, wobei Quantifizierbarkeit und die Empfindlichkeit von konventionellen RIAs oder ELISAs erreicht wird. Es wurde der Nachweis von Spurenkomponenten im Bereich von $10^{-12}$ mol/l bei benötigten Probemengen von $10^{-16}$ mol entsprechend z.B. ca. 1 pg bei Reaktionszeiten von weniger als 30 Minuten bei Raumtemperatur ermöglicht. Mit den beschriebenen Anordnungen können jedoch auch Tests geringerer Empfindlichkeitsanforderung ausgeführt werden. Es wurden Standardkurven über zwei bis drei Dekaden bei Auswertung mit dem Reflektometer Sanoquell® (fa. Quelle) erhalten. Die Chromatographiezeit für das Testelement einschließlich vollständiger Farbentwicklung liegt bei maximal 16 Minuten. Die Auswertung kann auch visuell erfolgen. Bei HCG als Analyt lag der Beginn des Meßbereichs in einem Beispiel mit einer an eine Festphase gebundenen Glucoseoxidase und einer Peroxidasemarkierung bei 0,3 nglml (entsprechend 3 U/l).

In dem folgenden Beispiel wird als konkrete Ausführungsform die Anwendung des Prinzips des kompetitiven Doppelantikörpertests vorgestellt. Bei dieser Testkonfiguration sind für die Bestimmungsreaktion und den Trennschritt vier Komponenten nacheinander in Reaktion zu bringen, wobei die Reaktionszeiten und die Konzentrationen des Reaktanden kritische Größen sind. Das Beispiel ist keinesweges als limitierend zu betrachten sondern dient lediglich der weiteren Erläuterung des Erfindungsgegenstandes.

**Übersicht I: Beispielhafte Testaufbauten mit Probe oder Probenvorverdünnung als Elutionsmittel**

Probe
O

Detektionszone

Saugzone

in V nachgewiesener Komplex

| Testprinzip | I | II | III | IV | V | VI | |
|---|---|---|---|---|---|---|---|
| kompetitiv, z.B. | | O—* | )ᵢ | | )₂ | *—O₁-)₂ | |
| | | O—* | | | )₁ | *—O₁ | |
| | | )ᵢ | O— | | )₂ | *—O₁-)₂ | |
| Glc, TMB | | O—* | )ᵢ GOD | | )₂ | *—O₁-)₂ | * = POD |
| Glc, TMB | | O—* | )ᵢ | GOD oder GOD—‍ | )₂ | *—O₁-)₂ | * 3 POD |
| TMB | | O—* | )ᵢ | Perborat )₂ | | *—O₁-)₂ | * = POD |
| Sandwich, z.B. | | )₁* | )₂ | | )₃ | *₁-O₂-)₃ | |
| immunome-trisch, z.B. | | )₁ | | | o‍‍ | *—O₁ | |

Symbolerklärung: vgl. Übersicht II

**Übersicht II: Beispielhafte Testaufbauten mit separatem Elutionsmittel**

Elutionsmittel

Probe

Detektionszone

Saugzone

| Testprinzip | I | II | III | IV | V | VI | |
|---|---|---|---|---|---|---|---|
| kompetitiv. z.B. | | O—* | o↓ | )ᵢ | )₂ | | *—O₁-)₂ |
| Sandwich, z.B. | | )₁* | o↓ | )₂ | )₃ | | *₁-O₂-)₃ |
| immunometrisch, z.B. | | | o↓ | )₁ | o‍‍ | | *—O |
| indirekter Antikörpernachweis | | )₁* | ≺↓ | | o‍‍ | | *—C—O |
| ELA (enzyme-labeled antigen) | | | ≺↓ | o—* | )‍‍ | | *—O—O |

GOG = Glucoseoxidase; POD = Peroxidase; TMB = Tetramethylbenzidin;
Glc = αD-Glucose
x↓ = Aufgabe der Komponente X auf die jeweilige Zone
o‍‍‍ = festphasengebundene Komponente
)‍ = bindende Komponente (Rezeptor)
≻ = Antikörper oder Rezeptor mit Bindungsstellen für einen anderen Rezeptor
* = Markierung; O = Komponente, die von einem Rezeptor gebunden werden kann

*Beispiel*

Vollintegrierter enzymimmunchemischer Teststreifen zum Nachweis von HCG mit eingebautem Chromogen-Substratsystem

### 1.1. Reagenzien

### 1.1.1. HCG-Peroxidase-Konjugat

HCG mit einer spezifischen Aktivität von ca. 3000 U/mg wurde von der Fa. Organon bezogen. Peroxidase aus Meerrettich wurde von der Fa. Boehringer Mannheim (Katalog Nr. 413 470) bezogen. Das heterobifunktionelle Reagenz N-γ-Maleimidobutyryloxy- succinimid (GMBS) wurde von der Fa. Behring Diagnostics bezogen und wie von Tanimori et al., 1983, in J. Imm. Meth. 62, 123-131, beschrieben, mit dem HCG umgesetzt. 2-Iminothiolan-hydrochlorid (Fa. Sigma, Kat.- r. I 6256) wurde wie von King et al., 1978, in Biochemistry 17, 1499-1506, beschrieben, mit Peroxidase umgesetzt. Aus dem GMBS-HCG und der Iminothiolan-Peroxidase wurde ein Konjugat wie von Tanimori et al. beschrieben hergestellt. Das Rohkonjugat wurde durch Gelchromatographie an Ultrogel® ACA 44 (Fa. LKB) gereinigt. Die Fraktion, in der etwa 1-2 Peroxidasemoleküle pro HCG-Molekül gekoppelt waren, wurde für den Test verwendet. Das Konjugat wurde mit Enzygnost® IgE Inkubationsmedium der Behringwerke, Best o. OS D, im weiteren kurz als Inkubationsmedium bezeichnet, verdünnt.

### 1.1.2. Antikörper

Antikörper gegen HCG wurden durch Immunisieren von Kaninchen und Antikörper gegen Kaninchen-IgG durch Immunisieren von Ziegen erhalten. Die IgG-Fraktionen wurden aus Serum durch Ammoniumsulfatfällung und Anionenaustauscherchromatographie gewonnen und durch Immunadsorption weiter gereinigt. Die angewandten Methoden sind beschrieben in dem Buch « Immunologische Arbeitsmethoden », Helmut Friemel, Herausgeber, 1984, Gustav Fischer Verlag, Stuttgart. Der Anti-HCG-Antiköper wurde in dem oben angegebenen Konjugatverdünnungspuffer endverdünnt.

### 1.1.3. Glucoseoxidase

Glucoseoxidase aus Aspergillus niger wurde als Lösung mit 300 U/mg bezogen (Fa. Serva, Kat.-No. 22737). Die Glucoseoxidase wurde mit Inkubationsmedium endverdünnt.

### 1.1.4. Glucose, Tetramethylbenzidin

α-D-Glucose und Tetramethylbenzidin-hydrochlorid wurden von der Fa. Serva, Kat.-Nr. 22720 bzw. 35926, bezogen.

### 1.2. Herstellung des Mittels

Die flächenförmigen Funktionsbereiche wurden wie folgt hergestellt:

Zur Herstellung der Elutionsmittelauftragzone wurde ein Vliesschwammtuch der Fa. Kalle, das ein trocken gepreßter Kunstschwamm aus regenerierter Cellulose ist, in einer Grösse von 20x6 mm zugeschnitten. Es wurde mit einer Lösung von 50 mg Glucose und 0,75 mg Tetramethylbenzidin-hydrochlorid je ml Wasser getränkt und im Luftstrom getrocknet.

Konjugat, Anti-HCG-Antikörper und Glucoseoxidase (je 5 μl mit 25 μl/ml, 100 μl/ml bzw. 0,1 mg/ml) wurden in gleichmäßigem Abstand auf ein 45x5 mm-Stück MN Nr. 1-Papier (Macherey & Nagel) hintereinander aufgetragen und an der Luft eingetrocknet.

Als Festphasenzone wurde ein 5 x 5 mm großes Stück Nr. 597-Papier der Fa. Schleicher & Schüll mit Anti-Kaninchen-IgG-Antikörper kovalent beschichtet. Dazu wurde der Antikörper an das mit Bromcyanaktivierte Papier, wie von Clarke et al., 1979, Meth. Enzymology, Vol. 68, 441-442, beschrieben, gekuppelt.

Als Saugzone wurde ein 20 x 5 mm-Stück Nr. 2668/8-Papier (Fa. Schleicher & Schüll) verwendet.

Die vier Papiere wurden 0,5-1 mm überlappend hintereinander mit Hilfe eines doppelseitigen Klebebandes (Testband der Fa. Beiersdorf) auf einer festen Unterlage fixiert, so daß ein 5 mm breiter Teststreifen entstand.

1.3. Testdurchführung

Zur Durchführung des Tests wurden jeweils 200μl einer HCG-Verdünnung in Inkubationsmedium auf das Vlies aufgetragen.

1.4. Ergebnis

Die chromatographische Entwicklung des Testelementes und die selbsttätig aublaufende Farbentwicklung waren nach 15 Minuten bei Raumtemperatur abgeschlossen und es konnte visuell oder mit einem Reflektometer ausgewertet werden.

Bei Auswertung der Festphasenzone (Nr. 597 - Papier) mit dem Blutglucoseauswertegerät Sanoquell der Fa. Quelle ergaben sich folgende Werte:

| HCG-Konzentration (U/l) | Meßwerte (mg Glucose pro dl Blut) |
| --- | --- |
| 0,3 | 107 |
| 3 | 117 |
| 30 | 95 |
| 300 | 70 |
| 3000 | 0 |

Mit dem Urinteststreifenauswertegerät Rapimat der Behringwerke wurden bei den gleichen Teststreifen folgende Werte erhalten:

| HCG-Konzentration (U/l) | Meßwerte (BIT) |
| --- | --- |
| 0,3 | 76 |
| 3 | 76 |
| 30 | 94 |
| 300 | 119 |
| 3000 | 135 |

Der hier gezeigte Teststreifenaufbau läßt sich auch realisieren, wenn die Glucoseoxidase und der Anti-HCG-Antikörper sich in der gleichen Zone befinden. Der entsprechend kürzere Teststreifen hat dann eine Testzeit von ca. 10 Minuten.

**Patentansprüche**

1. Analytisches Mittel zum Nachweis oder zur Bestimmung einer Komponente eines bioaffinen Bindungspaares (Analyt) in einer Flüssigkeit, bestehend aus mehreren flächenförmigen Zonen, die so hintereinander angeordnet sind, daß sie über ihre Kanten miteinander in saugfähigem Kontakt stehen, und daß sie zusammen mit der festen Unterlage ein flächenförmiges chromatographisches analytisches Mittel darstellen, enthaltend eine Elutionsmittelauftragszone (EMAZ) am einen Ende und eine Saugzone (SZ) am anderen Ende des Mittels sowie dazwischenliegende weitere saugfähige Zonen, in denen zu bioaffinen Wechselwirkungen mit dem Analyten befähigte Reaktionsteilnehmer so angeordnet sind, daß miteinander reaktionsfähige Reaktionsteilnehmer räumlich getrennt vorliegen, wobei ein Reaktand kovalent oder adsorptiv über eine bioaffine Wechselwirkung in einer zwischen der EMAZ und SZ und mit der SZ in Kontakt befindlichen Zone, der Festphasenzone (FPZ), fixiert ist oder in einer im Mittel ablaufenden Reaktion durch einen kovalent oder adsorptiv oder über eine bioaffine Wechselwirkung in der FPZ fixierten weiteren Reaktanden gebunden wird und wobei die Analytauftragzone die EMAZ oder eine Zone zwischen EMAZ und SZ ist, und ein markierter Reaktand sich in einer Zone zwischen der EMAX und FPZ nicht gebunden befindet, dadurch gekennzeichnet, daß die flächenförmigen Zonen aus Streifen gebildet sind, die aus unterschiedliche Materialien bestehen, wobei jeder Streifen auf der festen Unterlage fixiert ist.

2. Flächenförmiges diagnostisches Mittel nach Anspruch 1 , zum Nachweis von zwei oder mehr Analyten mit jeweils einer oder mehreren bioaffinen Bindungsstellen in einer Lösung, dadurch gekennzeichnet, daß es je Analyt eine räumlich getrennte Festphasenzone enthält, die mit trägergebundenen, für den jeweiligen Analyten spezifischen Bindungspartnern versehen ist und in dem die Analyten getrennt nachgewiesen werden.

3. Mittel nach Anspruch 1 oder 2, dadurch gekennzeichnet, daß die EMAZ die Funktion eines Volumendosierelementes hat und mindestens so viel Flüssigkeit an die folgenden Zonen abgibt, daß die Flüssigkeit, durch Kapillarkräfte gesteuert, bis zum Ende der SZ gelangt.

4. Mittel nach einem der Ansprüche 1 bis 3, dadurch gekennzeichnet, daß die EMAZ ein Kunststoffschwamm oder eine partikuläre Schicht, bestehend aus hydrophilen Polymeren, ist, die gegebenenfalls Chemikalien, Puffersubstanzen oder andere testnotwendige Substanzen enthalten kann.

5. Mittel nach einem der Ansprüche 1 bis 4, dadurch gekennzeichnet, daß die Probenauftragzone Blutzellen zurückhält.

6. Mittel nach einem der Ansprüche 1 bis 5, dadurch gekennzeichnet, daß die Probenauftragzone auf eine der flächenförmigen Zonen des chromatographierenden Teils des Mittels auflaminiert ist und mit dieser in saugfähigem Kontakt steht.

7. Mittel nach einem der Ansprüche 1 bis 6, dadurch gekennzeichnet, daß alle oder ein Teil der zum Nachweis der Markierung notwendigen Reagenzien in einer oder mehreren der flächenförmigen Zonen des Mittels oder in einer Zone, die auf eine der flächenförmigen Zonen des chromatographierenden Teils des Mittels auflaminiert ist und mit dieser in saugfähigem Kontakt steht, enthalten sind.

8. Verfahren unter Verwendung eines Mittels nach einem der Ansprüche 1 bis 7, dadurch gekennzeichnet, daß alle für den Ablauf der Reaktion erforderlichen, im Mittel vorhandenen Reaktanden in dehydratisierter Form vorliegen und durch die dem Mittel zugeführten Flüssigkeiten rehydratisiert oder solvatisiert werden.

9. Verfahren nach Anspruch 8, unter Verwendug eines Mittels nach einem der Anprüche 1 bis 7, dadurch gekennzeichnet, daß nach Aufgabe der den Analyten enthaltenden flüssigen Probe auf die EMAZ oder nach Aufgabe der Probe auf eine Probenauftragzone und Aufgabe eines Elutionsmittels auf die EMAZ die Flüssigkeit bis zum Ende der SZ, durch Kapillarkräfte gesteuert, gelangt und dadurch Reaktionen zwischen im Mittel enthaltenen Reaktionsteilnehmern und dem Analyten in Gang gesetzt werden und nach chromatographischem Abtrennen der nicht spezifisch an die Festphase gebundenen Markierung die Menge der Markierung in der Festphasenzone, die ein Maß für die Analytkonzentration in der Probe ist, bestimmt wird.

10. Verfahren nach Anspruch 8 oder 9 unter Verwendug eines Mittels nach einem der Anprüche 1 bis 7, dadurch gekennzeichnet, daß den im Mittel ablaufenden Reaktionen die Prinzipien der immunologischen Nachweisreaktionen des kompetitiven immunometrischen oder Sandwich-Immunoassays oder des indirekten Antikörpernachweises mit markiertem Antikörper oder des Antiköpernachweises mit markiertem Antigen zugrundeliegen.

11. Verfahren nach einem der Ansprüche 8, 9 oder 10 unter Verwendug eines Mittels nach einem der Anprüche 1 bis 7, dadurch gekennzeichnet, daß das Markierungsmittel ein Fluorophor ist, der direkt nachgewiesen oder gemessen oder nach Zugabe eines im Mittel vorhandenen Reagenzes nachgewiesen oder gemessen wird oder daß aus dem Markierungsmittel durch Zugabe eines im Mittel vorhandenen Reagenzes ein Fluorophor gebildet wird, der direkt oder nach Zugabe eines weiteren Reagenzes nachgewiesen oder gemessen wird.

12. Verfahren nach einem der Ansprüche 8 bis 10 unter Verwendug eines Mittels nach einem der Ansprüche 1 bis 7, dadurch gekennzeichnet, daß das Markierungsmittel eine zur Chemilumineszenz anregbare Verbindung ist, wobei nach Zugabe eines im Mittel vorhandenen Reagenzes eine Chemilumineszenz nachgewiesen oder gemessen werden kann.

13. Verfahren nach den Ansprüchen 8, 9 oder 10 unter Verwendug eines Mittels nach einem der Anprüche 1 bis 7, dadurch gekennzeichnet, daß das Markierungsmittel ein Enzym ist, dessen Aktivität unter Zuhilfenahme eines im Mittel vorhandenen Reagenzes bestimmt wird.

## Claims

1. An analytical device for the detection or determination of a component of a binding pair having biological affinity (analyte) in a fluid, said device being composed of several sheet-like zones which are arranged behind one another in such a manner that they are in absorbent contact with one another through their edges and that they form, together with the solid support, a sheet-like chromatographic analytical device, containing a mobile phase application zone (MPAZ) at one end of the device and an absorption zone (AZ) at the other end and also further absorptive zones situated intermediately in which reactants capable of interactions, of biological affinity, with the analyte are arranged in such a way that reactants capable of reacting with one another are present, separated spatially, in which a reactant is fixed to the solid phase zone (SPZ) by means of covalent bonds or adsorptively or via an interaction of biological affinity in a zone which is located between the MPAZ and AZ and is in contact with the AZ, or becomes attached in a reaction which takes place in the device through a further reactant which is fixed in the SPZ by covalent bonds or adsorptively or via an interaction of biological affinity, and in which the analyte application zone is the MPAZ or a zone between MPAZ and AZ, and a labeled reactant is located, unattached, in a zone between the MPAZ and the SPZ, wherein the sheet-like zones are formed from strips comprising different materials, each strip being fixed to the solid support.

2. A sheet-like diagnostic device as claimed in claim 1 for the detection in a solution of two or more analytes each of which has one or more attachment points of biological affinity, which contains, per analyte, a spatially separated solid phase zone which is provided with combination partners Attached to the support and specific for the particular analyte, and in which device the analytes are detected separately.

3. A device as claimed in claim 1 or 2, wherein the MPAZ has the function of a volume metering element and releases to the subsequent zones at least sufficient liquid for the liquid, controlled by capillary forces, to reach the end of the AZ.

4. A device as claimed in one of claims 1 to 3, wherein the MPAZ is a plastic sponge or a particulate layer which is composed of hydrophilic polymers and which can, if appropriate, contain chemicals, buffer substances or other substances required for the test.

5. A device as claimed in one of claims 1 to 4, wherein the sample application zone retains blood cells.

6. A device as claimed in one of claims 1 to 5, wherein the sample application zone is laminated onto one of the sheet-like zones of the chromatographing section of the device and is in absorptive contact with this zone.

7. A device as claimed in one of claims 1 to 6, wherein all or some of the reagents required for the detection of the labeling are present in one or more of the sheet-like zones of the device or in a zone which is laminated onto one of the sheet-like zones of the chromatographing section of the device and is in absorptive contact with this zone.

8. A process using a device as claimed in one of claims 1 to 7, wherein all the reactants present in the device and necessary for the reaction to take place are in a dehydrated form and are rehydrated or solvated by the liquids fed to the device.

9. The process as claimed in claim 8, using a device according to one of claims 1 to 7, wherein, after the liquid sample containing the analyte has been fed to the MPAZ or after the sample has been fed to the sample application zone and a mobile phase has been fed to the MPAZ, the liquid reaches the end of the AZ, under the control of capillary forces, and reactions between reactants contained in the device and the analyte are thereby set in operation, and, after the labeling which is not specifically attached to the solid phase has been removed chromatographically, the amount of the labeling in the solid phase zone, which is a measure of the analyte concentration in the sample, is determined.

10. The process as claimed in claim 8 or 9, using a device as claimed in one of claims 1 to 7, wherein the reactions taking place in the device are based on the principles of immunological detection reactions, of competitive immunometric or sandwich immunoassay or of indirect antibody detection by means of a labeled antibody or of antibody detection by means of a labeled antigen.

11. The process as claimed in one of claims 8, 9 or 10, using a device as claimed in one of claims 1 to 7, wherein the labeling agent is a fluorophore which is detected or measured directly or is detected or measured after the addition of a reagent present in the device, or a fluorophore which is detected or measured directly or after the addition of a further reagent is formed from the labeling agent by the addition of a reagent present in the device.

12. The process as claimed in one of claims 8 to 10, using a device as claimed in one of claims 1 to 7, wherein the labeling agent is a compound which can be excited to give chemiluminescence, it being possible to detect or measure chemiluminescence after the addition of a reagent present in the device.

13. The process as claimed in claims 8, 9 or 10, using a device as claimed in one of claims 1 to 7, wherein the labeling agent is an enzyme the activity of which is determined with the aid of a reagent present in the device.

## Revendications

1. Dispositif analytique pour la mise en évidence ou pour la détermination, dans un liquide, d'un composant d'une paire de corps liés par bioaffinité (corps à analyser), constitué de plusieurs zones planes qui sont disposées les unes derrière les autres de manière a être en contact absorbant les unes avec les autres par leurs bords, et à constituer, conjointement avec le support solide, un dispositif analytique chromatographique plan contenant une zone d'introduction d'éluant (ZIE) à une extrémité et une zone d'absorption (ZA) à l'autre extrémité du dispositif, ainsi que d'autres zones absorbantes intercalées, dans lesquelles des partenaires réactionnels capables d'interactions de bioaffinité avec les produits à analyser sont disposés de manière que des partenaires réactionnels réactifs les uns avec les autres se trouvent séparés dans l'espace, un partenaire réactionnel étant fixé par covalence ou adsorption, par l'intermédiaire d'une interaction de bioaffinité dans une zone, la zone de phase solide (ZPS) qui se trouve entre la ZIE et la ZA et en contact avec la ZA, ou étant lié dans une réaction se déroulant dans le dispositif, au moyen d'un autre partenaire réactionnel fixé par covalence ou adsorption ou par l'intermédiaire d'une interaction de bioaffinité dans la ZPS, et la zone d'introduction du produit à analyser étant la ZIE ou une zone située entre la ZIE et la ZA, et un partenaire réactionnel marqué se trouvant, non lié, dans une zone située entre la ZIE et la ZPS, caractérisé en ce que les zones planes consistent en bandes qui sont constituées de matériaux différents, chaque bande étant fixée sur le support solide.

2. Dispositif diagnostique plan selon la revendication 1, pour la mise en évidence de deux corps à analyser ou plus, comportant chacun un ou plusieurs sites de liaison par bioaffinité, dans une solution, caractérisé en ce qu'il contient, pour chaque corps à analyser, une zone de phase solide séparée dans l'espace, qui est munie de partenaires de liaison fixés sur support, spécifiques de chaque corps à analyser, et dans laquelle les corps à analyser sont détectés séparément.

3. Dispositif selon la revendication 1 ou 2, caractérisé en ce que la ZIE a la fonction d'un élément doseur de volume et fournit aux zones suivantes une quantité de liquide suffisante pour que le liquide, entraîné par des forces de capillarité, arrive jusqu'à l'extrémité de la ZA.

4. Dispositif selon l'une des revendications 1 à 3, caractérisé en ce que la ZIE est une éponge de matière plastique ou une couche particulaire composée de polymères hydrophiles, qui peut éventuellement contenir des produits chimiques, des substances tampons ou d'autres substances requises.

5. Dispositif selon l'une des revendications 1 à 4, caractérisé en ce que la zone d'introduction d'échantillon retient les globules sanguins.

6. Dispositif selon l'une des revendications 1 à 5, caractérisé en ce que la zone d'introduction d'échantillon est appliquée par laminage sur l'une des zones planes de la partie chromatographique du dispositif et est en contact absorbant avec celle-ci.

7. Dispositif selon l'une des revendications 1 à 6, caractérisé en ce que la totalité ou une partie des réactifs requis pour la détection du marquage est contenue dans une ou plusieurs des zones planes du dispositif ou dans une zone qui est appliquée par laminage sur l'une des zones planes de la section chromatographique du dispositif et est en contact absorbant avec celle-ci.

8. Procédé utilisant un dispositif selon l'une des revendications 1 à 7, caractérisé en ce que tous les réactifs requis pour le déroulement de la réaction, qui sont présents dans le dispositif, se trouvent sous forme déshydratée et sont solvatés ou réhydratés par les liquides envoyés au dispositif.

9. Procédé selon la revendication 8, utilisant un dispositif selon l'une des revendications 1 à 7, caractérisé en ce que, après application sur la ZIE de l'échantillon liquide contenant les corps à analyser, ou après application de l'échantillon sur une zone d'introduction d'échantillon et application d'un éluant sur la ZIE, le liquide, entraîné par des forces de capillarité, arrive jusqu'à l'extrémité de la ZA, et des réactions sont ainsi déclenchées entre des partenaires réactionnels contenus dans le dispositif et le corps à analyser et, après séparation chromatographique du marquage non spécifiquement lié à la phase solide, la quantité du marquage dans la zone de phase solide, qui est une mesure de la concentration du corps à analyser dans l'échantillon, est déterminée.

10. Procédé selon la revendication 8 ou 9, utilisant un dispositif selon l'une des revendications 1 à 7, caractérisé en ce que les principes des réactions de dosage immunologique de l'essai immunologique en sandwich ou immunométrique compétitif ou de la détection indirecte d'anticorps à l'aide d'anticorps marqué ou de la détection d'anticorps à l'aide d'un antigène marqué sont à la base des réactions qui se déroulent dans le dispositif.

11. Procédé selon l'une des revendication 8, 9 ou 10, utilisant un dispositif selon l'une des revendications 1 à 7, caractérisé en ce que le marqueur est un fluorophore qui est détecté ou mesuré directement ou détecté ou mesuré après addition d'un réactif présent dans le dispositif, ou en ce que, à partir du marqueur est formé, par addition d'un réactif présent dans le dispositif, un fluorophore qui est détecté ou mesuré directement ou après addition d'un autre réactif.

12. Procédé selon l'une des revendications 8 à 10, utilisant un dispositif selon l'une des revendications 1 à 7, caractérisé en ce que le marqueur est un composé qui sur excitation donne lieu à de la chimiluminescence, ce par quoi, après addition d'un réactif présent dans le dispositif, une chimiluminescence peut être détectée ou mesurée.

13. Procédé selon la revendication 8, 9 ou 10, utilisant un dispositif selon l'une des revendications 1 à 7, caractérisé en ce que le marqueur est une enzyme dont l'activité est déterminée à l'aide d'un réactif présent dans le dispositif.